# EUROPÄISCHE PATENTSCHRIFT

(11) **EP 1 045 721 B1**
(45) Veröffentlichungstag und Bekanntmachung des Hinweises auf die Patenterteilung: **15.06.2005**
(21) Anmeldenummer: 99907357.0
(22) Anmeldetag: 08.01.1999
(51) Int. Cl.: A61N 7/00

(54) **VORRICHTUNG ZUR BEHANDLUNG VON KÖRPERGEWEBE, INSBESONDERE VON OBERFLÄCHENNAHEM WEICHGEWEBE, MITTELS ULTRASCHALL**
DEVICE FOR TREATING BODY TISSUE, ESPECIALLY SOFT TISSUE LOCATED NEAR THE SURFACE, BY MEANS OF ULTRASOUND
DISPOSITIF POUR LE TRAITEMENT PAR ULTRASONS DE TISSUS DU CORPS, EN PARTICULIER DE TISSUS MOUS PROCHES DE LA SURFACE

(30) Priorität: 08.01.1998 DE 19800416
(43) Veröffentlichungstag der Anmeldung: 25.10.2000
(73) Patentinhaber: Karl Storz GmbH & Co. KG, 78532 Tuttlingen (DE)
(72) Erfinder: IRION, Klaus M., 78576 Liptingen (DE); ISSE, Nicanor G., Burbank, CA 91502 (US)
(74) Vertreter: Heuckeroth, Volker, Dr.
(86) Internationale Anmeldenummer: PCT/EP1999/000074
(87) Internationale Veröffentlichungsnummer: WO 1999/034870

(56) Entgegenhaltungen:
- EP-A- 0 459 239
- EP-A- 0 614 651
- EP-A- 0 668 052
- US-A- 5 003 965
- US-A- 5 139 496
- US-A- 5 601 526

## Beschreibung

Die Erfindung betrifft eine Vorrichtung zur Behandlung von Körpergewebe, insbesondere von oberflächennahem Weichgewebe, mittels Ultraschall, mit einer Ultraschall-Erzeugungseinrichtung und mit einem Applikator, mit dem der Ultraschall über eine der Körperoberfläche zugewandte Applikatorfläche von außen durch die Körperoberfläche in das Körpergewebe einkoppelbar ist, und mit einer Ansaugvorrichtung zum Ansaugen der Körperoberfläche an den Applikator, wobei die Ansaugvorrichtung eine Saugvorrichtung aufweist, mit der der Applikator über zumindest einen Saugkanal in Verbindung steht, und wobei die Applikatorfläche nach innen gewölbt ausgebildet ist und die Auswölbung der Applikatorfläche nach innen zum Ansaugen der Körperoberfläche bis an die Applikatorfläche dient.

Eine derartige Vorrichtung ist aus dem Dokument EP 0 614 651 A1 bekannt.

Die Anwendung von Ultraschall zu Heilzwecken hat eine erhöhte Bedeutung gewonnen. Der Ultraschall wird je nach Therapie in Form von kontinuierlichen oder gepulsten Ultraschallfeldern appliziert.

Mit Erfolg wurde Ultraschall bei der sogenannten extrakorporalen Stoßwellentherapie sowie bei der Ultraschallwellentherapie zur Behandlung von Steinleiden in Niere, Urether, Galle usw. eingesetzt. Eine Vorrichtung zur Behandlung von Körpersteinen, ein sogenannter Lithotriptor, ist bspw. aus dem CH-Firmenprospekt der Storz Medical AG, Kreuzlingen, Schweiz, "STORZ MODULITH®-Systeme für die extrakorporale Lithotripsie", bekannt. Mit diesem Lithotriptor werden kurze Ultraschall-Pulse extrakorporal, d.h. außerhalb des Körpers, erzeugt, in den Körper eingekoppelt und auf die Körpersteine im Körper fokussiert. Zuvor werden die Körpersteine über Ultraschall-Schnittbildgeräte oder über Röntgensysteme lokalisiert.

Zur extrakorporalen Behandlung von Tumoren sind Entwicklungen im Gange, mit Hilfe spezieller Ultraschallapplikatoren Ultraschallwellen zu erzeugen und von außen in den Tumor einzukoppeln. Durch speziell dosierte Einwirkzeiten des im Tumor wirkenden Ultraschalls kommt es zu einer Überwärmung des Tumorgewebes und somit zu dessen Zerstörung.

Aber nicht nur die thermische Wirkung des Ultraschalls führt zur Auflösung von Zellvexbänden, sondern auch die rein mechanische Wirkung in Form von Kavitation. Schallwellen breiten sich in einem Medium als sich periodisch ändernde Dichteschwankungen des Mediums aus. Ein Volumenelement des Mediums wird abwechselnd verdichtet (Überdruck) und expandiert (Unterdruck). Der Unterdruck kann in einer Flüssigkeit, bspw. in der Zellflüssigkeit, zur Dampfblasenbildung führen, wodurch schließlich die Zellwände und/oder der Zellverband zerstört werden.

Es hat sich gezeigt, daß diese Wirkung des Ultraschalls geeignet ist, Weichgewebe, bspw. Fettgewebe, zu desintegrieren, d.h. aufzulösen. Eine aus der US-Firmenschrift der Wells Johnson Company, Tucson, Arizona, USA, "Introducing SILBERG E.U.S.™ External Ultrasonic System", bekannte Vorrichtung wird dazu verwendet, oberflächennahes Fettgewebe abzutragen. Dazu weist die bekannte Vorrichtung einen Applikator auf, mittels dem der Ultraschall von außen durch die Körperoberfläche auf das darunterliegende Weichgewebe eingekoppelt wird.

Ein Nachteil dieser bekannten Vorrichtung besteht in der Schwierigkeit, über die gesamte aktive Applikatorfläche eine gleichmäßig gute Ankopplung des Applikators an das zu behandelnde Gewebe zu erzielen. Ist dies nicht der Fall, so wird nicht nur die eingebrachte Schallenergie nicht optimal genutzt, sondern es kommt auch zu übermäßiger Hitzeentwicklung.

Ein weiterer Nachteil dieser bekannten Vorrichtung besteht darin, daß es nicht möglich ist, die Tiefenwirkung des Ultraschalls, d.h. die Eindringtiefe des wirksamen Ultraschalls bzw. dessen Fokus zu kontrollieren. Durch das Andrücken des Applikators kann es zu einer Verdichtung bzw. Verdrängung des darunterliegenden Gewebes kommen, so daß die einzelnen Gewebeschichten nicht mehr die ursprüngliche Dicke bzw. Lage haben. Durch die fehlende Tiefenkontrolle ist es somit nicht möglich, den Ultraschall gezielt in das zu behandelnde Körpergewebe einzukoppeln. Es besteht dabei die Gefahr, daß nicht zu behandelndes Körpergewebe durch den Ultraschall geschädigt wird.

Die aus dem eingangs genannten Dokument bekannte Vorrichtung weist einen Applikator auf, dessen Applikatorfläche nach innen gewölbt ausgebildet ist, wobei der durch die Wölbung aufgespannte Raum, der durch die Applikatorfläche auf der einen Seite begrenzt ist, mit einem mit Flüssigkeit gefüllten Kissen ausgefüllt ist, um die Einkopplung des Ultraschalls in die Körperoberfläche zu verbessern und eine Kühlung der Haut zu ermöglichen. Des Weiteren ist bei dieser Vorrichtung eine Ansaugvorrichtung vorgesehen, die eine Saugpumpe umfaßt, die über eine Saugleitung mit dem Applikator verbunden ist, wobei die Saugleitung im Randbereich außerhalb der Applikatorfläche in den Applikator mündet. Des Weiteren ragt bei diesem Applikator die Ultraschallerzeugungseinheit in den von der Wölbung des Applikators aufgespannten Raum hinein.

Eine weitere Vorrichtung zur Behandlung von oberflächennahem Körpergewebe mittels Ultraschall der eingangs genannten Art ist aus der US-A-5 618 275 bekannt. Bei dieser Vorrichtung weist der Applikator ein Gehäuse auf, in das ein Ultraschallwandler eingebettet ist. An dem Gehäuse ist der Körperoberfläche zugewandt eine Manschette vorgesehen, die zusammen mit dem Gehäuse eine Kammer eines vorbestimmten Volumens definiert, in der eine auf die Körperoberfläche zu applizierende Substanz zwischen der Körperoberfläche und der Applikatorfläche festgehalten wird, so daß die Substanz nicht aus der durch die Manschette gebildete Kammer entweichen kann. Die Manschette ist aus Gummi oder dgl. flexibel ausgebildet. Die durch die Manschette zwischen der Applikatorfläche und der Körperoberfläche gehaltene therapeutische Substanz soll mittels der Ultraschallbehandlung leichter in das zu behandelnde Gewebe eindringen können.

Der Erfindung liegt somit die Aufgabe zugrunde, eine Vorrichtung der eingangs genannten Art dahingehend weiterzubilden, daß der Ultraschall gezielt in das zu behandelnde Körpergewebe eingekoppelt werden kann und eine Tiefenkontrolle der Ultraschalltherapie ermöglicht wird.

Erfindungsgemäß wird die der Erfindung zugrundeliegende Aufgabe dadurch gelöst, daß der zumindest eine Saugkanal in der Applikatorfläche mündet.

Durch das Ansaugen der Körperoberfläche wird auch das darunterliegende Körpergewebe an den Applikator gesaugt, wodurch eine Fixierung des Gewebes an dem Applikator erreicht wird. Durch die Fixierung des Gewebes an dem Applikator kann der Ultraschall zur Therapie optimal flächig in das Gewebe eingekoppelt werden. Durch das Ansaugen des Gewebes wird der Behandlungsbereich im Körper entfernungsmäßig festgelegt. Somit kann der Ultraschall gezielt und kontrolliert in das Körpergewebe eingekoppelt werden, weil der Abstand zwischen der Austrittsstelle des Ultraschalls aus dem Applikator und dem Körpergewebe definiert ist. Eine Ansaugung der Körperoberfläche und damit des zu behandelnden darunterliegenden Körpergewebes hat gegenüber einem Anpressen des Applikators an die Körperoberfläche den vorteil, daß beim Ansaugen das unter der Körperoberfläche liegende Weichgewebe nicht seitlich verdrängt wird, bzw. seitlich ausweicht, und sich somit im Bereich vor dem Applikator verdünnt, sondern gerade umgekehrt in den Einkoppelbereich des Ultraschalles gezogen wird.

Der Applikator weist zumindest einen in der Applikatorfläche mündenden Saugkanal auf, der mit der Saugvorrichtung verbunden ist.

Bei dieser Maßnahme kann auf vorteilhaft einfache Weise zwischen der Körperoberfläche und der Applikatorfläche ein Vakuum erzeugt werden, indem der in die Applikatorfläche mündende Saugkanal durch die Saugvorrichtung mit einem Unterdruck beaufschlagt wird, wodurch die Körperoberfläche an die Applikatorfläche angesaugt wird. Durch das vorsehen einer Mehrzahl von in der Applikatorfläche mündenden Saugkanälen kann die Ansaugung der Körperoberfläche weiter verbessert werden. Wird der Applikator mit nur einem Saugkanal ausgebildet, so ist es vorteilhaft, wenn der Saugkanal etwa mittig in der Applikatorfläche mündet. Der Saugkanal kann auch mit einer oder mehreren oberflächlich offenen Kanälen oder Rillen in der Applikatorfläche verbunden sein.

In einer bevorzugten Weiterbildung der Erfindung ist der Ultraschall mittels des Applikators fokussiert in das Körpergewebe einkoppelbar.

Durch das fokussierte Einkoppeln des Ultraschalls in das Körpergewebe kann der Ultraschall in einem kleinen Volumenelement von wenigen Millimetern Ausdehnung konzentriert werden. Durch die Konzentration des Ultraschalls wird die Leistungsdichte des Ultraschalls im Fokus erhöht, wodurch die therapeutische Wirkung, nämlich die Desintegration des Weichgewebes, begünstigt wird. Zum anderen kann dadurch insgesamt mit einer geringeren Energiedichte des Ultraschalls gearbeitet werden, wodurch der weitere Vorteil entsteht, daß die Energiedichte an der Körperoberfläche reduziert wird und eine übermäßige Erwärmung der Dermis vermieden wird. Eine fokussierte Einkoppelung des Ultraschalls in das Körpergewebe ist mit der aus der eingangs genannten US-Firmenschrift bekannten Vorrichtung nicht möglich.

Eine von der Form der Applikatoroberfläche weitgehend unabhängige Fokussiermöglichkeit besteht z.B. in der zeitlich definierten Ansteuerung der einzelnen Schwingerelemente des Applikators (Phased-Array-Technik).

Die Applikatorfläche ist nach innen gewölbt ausgebildet.

Diese Maßnahme hat den Vorteil, daß das Körpergewebe zumindest teilweise in die Auswölbung gesaugt wird. Je nach Saugwirkung kann das Körpergewebe jedoch auch soweit in die Auswölbung gesaugt werden, daß die Körperoberfläche an der Applikatorfläche anliegt. Insbesondere bei der Anwendung der erfindungsgemäßen Vorrichtung zur Desintegration von oberflächennahem Weichgewebe ergibt sich ein weiterer Vorteil daraus, daß oberflächennahes Weichgewebe, d.h. insbesondere Epidermis, Dermis und Fettgewebe, bevorzugt in die Auswölbung gesaugt wird, während das sich an das oberflächennahe Weichgewebe anschließende Muskelgewebe aufgrund seiner höheren Festigkeit weniger gut folgen kann. Ein weiterer Vorteil der nach innen gewölbten Ausbildung der Applikatorfläche besteht darin, daß die gewölbte Applikatorfläche eine geometriebedingte Fokussierung des Ultraschalls bewirken kann. Ein weiterer Vorteil dieser Maßnahme besteht darin, daß die Gewebeoberfläche, d.h. die Oberfläche der Haut und auch die etwaiger Gewebegrenzflächen, im angesaugten Zustand in der Regel größer ist als im nicht angesaugten, so daß die Schallintensität und damit die unerwünschte Erwärmung dort reduziert wird.

In einer ersten bevorzugten Ausgestaltung ist die Applikatorfläche etwa radialsymmetrisch kalottenförmig ausgebildet.

Mit dieser Ausgestaltung der Applikatorfläche kann eine Fokussierung bzw. Konzentration des Ultraschalls in einem kleinen Volumen im Körpergewebe erreicht werden. Durch die Ansaugung des Körpergewebes an den Applikator weist der Fokus eine wohl definierte Lage in dem Körpergewebe in einem wohl definierten Abstand von der Körperoberfläche auf.

In einer zweiten bevorzugten Ausgestaltung ist die Applikatorfläche etwa zylindersymmetrisch mit etwa U-förmigem Querschnitt ausgebildet.

Bei dieser Ausgestaltung der Applikatorfläche kann der in das Körpergewebe eingekoppelte Ultraschall über die Länge des Applikators entlang einer Linie in dem Körpergewebe fokussiert werden. Dadurch entsteht der Vorteil, daß gleichzeitig ein größeres längliches Areal des Körpergewebes behandelt werden kann.

Durch die Ansaugung des Körpergewebes ist gewährleistet, daß der Fokus des Ultraschalls entlang einer Linie in dem Körpergewebe über die Länge des Applikators wohldefiniert ist.

In einer weiteren bevorzugten Ausgestaltung der Erfindung weist die Ultraschall-Erzeugungseinheit ein elektrisch anregbares Schwingerelement oder mehrere elektrisch anregbare Schwingerelemente auf, das in der Applikatorfläche angeordnet ist bzw. die in der Applikatorfläche angeordnet sind.

Durch diese Maßnahme kann die Tiefenkontrolle der Ultraschalltherapie in Verbindung mit einem Ansaugen der Körperoberfläche an die Applikatorfläche weiter verbessert werden, da der Fokus des Ultraschalls durch das Ansaugen des Körpergewebes an das Schwingerelement im Körper entfernungsmäßig definiert festgelegt ist. Die Einkoppelung des Ultraschalls in das Körpergewebe wird dabei ebenfalls verbessert. Als Schwingerelemente können Ultraschallwandler in Form von piezo-keramischen Elementen verwendet werden, die Ultraschall mit der für therapeutische Zwecke erforderlichen Leistungsdichte und Frequenz erzeugen. Durch die Verwendung mehrerer Schwingerelemente, die entsprechend in der Applikatorfläche verteilt sind, kann bei einer gewölbten Ausbildung der Applikatorfläche vorteilhaft einfach eine geometriebedingte Fokussierung des Ultraschalls erreicht werden. Durch eine entsprechende Anordnung der Schwingerelemente bzw. durch eine entsprechende geometrische Ausbildung der Applikatorfläche kann auch gezielt eine leichte Defokussierung erreicht werden, um den Wirkungsbereich zu vergrößern und gleichzeitig eine Überhitzung des Gewebes zu verhindern.

Dabei ist es bevorzugt, wenn die Schwingerelemente mit unterschiedlichen Frequenzen und/oder mit unterschiedlichen Phasen angeregt sind.

Durch diese Maßnahme wird der Vorteil erzielt, daß durch die Anregung der einzelnen Schwingerelemente mit unterschiedlichen Frequenzen und/oder mit unterschiedlichen Phasen Lage und Ausdehnung des Fokus-Bereichs anstatt geometrisch elektronisch eingestellt werden können.

In einer weiteren bevorzugten Ausgestaltung der Erfindung steht der Applikator mit einer Saug- und Spülvorrichtung für ein Fluid in Verbindung, das zwischen die Applikatorfläche und die Körperoberfläche gebracht und wieder abgesaugt werden kann.

Die Einleitung des Fluids zwischen die Applikatorfläche und die Körperoberfläche während der Ultraschalltherapie hat den Vorteil, daß die Körperoberfläche durch das Fluid gekühlt werden kann. Auch bei einer Fokussierung des Ultraschalls im zu behandelnden Körpergewebe bewirkt der durch die Körperoberfläche eintretende Ultraschall noch eine geringe Erwärmung der Hautoberfläche. Diese Erwärmung kann durch das Fluid vermieden werden. Des weiteren hat das Einleiten des Fluids den Vorteil, daß das Fluid als Koppelmedium zwischen der Applikatorfläche und der Körperoberfläche wirkt und die Einkoppelung des Ultraschalls in den Körper verbessert.

Dabei ist es bevorzugt, wenn ein Absaugkanal für das Fluid mittig in der Applikatorfläche mündet.

Bei dieser Ausgestaltung kann das Fluid zentral an dem Koppelbereich abgesaugt werden. Andererseits kann dieser Absaugkanal bei Unterbrechung oder Drosselung der Zufuhr des Fluids in den Bereich zwischen die Applikatorfläche und der Körperoberfläche zur Ansaugung der Körperoberfläche an die Applikatorfläche verwendet werden, wodurch ein Kanal eingespart werden kann.

Weiterhin ist es dabei bevorzugt, wenn mindestens ein Einlaßkanal randseitig des Applikators in den Bereich zwischen die Applikatorfläche und die Körperoberfläche mündet.

Dieser Einlaßkanal kann auch ringförmig ausgebildet sein.

Diese Maßnahme hat in Verbindung mit der in der Applikatorfläche mittig vorgesehenen Absaugung den Vorteil, daß das randseitig in den Koppelbereich zwischen der Applikatorfläche und der Körperoberfläche einströmende Fluid in dem Koppelbereich gut verteilt werden kann.

In einer weiteren bevorzugten Ausgestaltung der erfindungsgemäßen Vorrichtung weist der Applikator randseitig eine Abdichtung gegenüber der Körperoberfläche auf.

Durch diese Maßnahme wird einerseits der Vorteil erzielt, daß die Saugwirkung zum Ansaugen des Körpergewebes verbessert werden kann, andererseits das Fluid sicher in dem Bereich zwischen der Applikatorfläche und der Körperoberfläche gehalten werden kann. Als Abdichtung kann bspw. ein am Umfang der Applikatorfläche vorgesehener Dichtwulst verwendet werden.

In einer weiteren bevorzugten Ausgestaltung ist die Tiefe des Fokus des Ultraschalls im Körpergewebe einstellbar.

Durch diese Maßnahme kann die Tiefenkontrolle der Ultraschalltherapie weiter verbessert werden und an die Dicke der zu behandelnden Gewebeschicht angepaßt werden. Als günstig erscheint eine Tiefe des fokus, die etwa der halben Dicke der Fettgewebeschicht entspricht. Durch die Einstellbarkeit wird darüber hinaus vorteilhaft vermieden, daß durch eine zu hohe Eindringtiefe des Ultraschalls sich an das Fettgewebe anschließendes Muskelgewebe durch den Ultraschall geschädigt wird.

In einer weiteren bevorzugten Ausgestaltung ist der Abstand zwischen dem bzw. den Schwingerelementen und der Körperoberfläche verstellbar.

Da die Körperoberfläche durch das Ansaugen im wesentlichen an der Applikatorfläche anliegt oder sich in deren unmittelbarer Nähe befindet, stellt diese Maßnahme eine vorteilhafte Möglichkeit dar, die Tiefe des Fokus des Ultraschalls im Körpergewebe einzustellen.

Weiterhin ist es bevorzugt, wenn an dem Applikator ein Abstandshalter befestigbar ist.

Der Abstandshalter stellt eine vorteilhaft einfache Möglichkeit dar, die Eindringtiefe des Ultraschalls in das Körpergewebe zu verändern. Außerdem kann die Auswölbung des Applikators durch den Abstandshalter effektiv vergrößert werden, so daß mehr Gewebe in die Auswölbung gesaugt werden kann. Bei einem Applikator mit ebener Applikatorfläche kann sogar bei eingebrachtem Abstandshalter ein Saugraum gebildet werden. Der Abstandshalter kann vorteilhafterweise gleichzeitig die Funktion der zuvor erwähnten Abdichtung gegenüber der Körperoberfläche ausüben.

In einer weiteren bevorzugten Ausgestaltung beträgt der Abstand des Fokus von der Applikatorfläche zwischen 5 und 40 mm.

Bei diesem Abstand des Fokus von der Applikatorfläche kann vorteilhaft die gesamte Dicke der oberflächennahen Weichgewebeschicht (Epidermis, Dermis, Fettgewebe) behandelt werden.

In einer weiteren Ausgestaltung weist der Applikator zumindest ein Ultraschall-Empfängerelement auf, das reflektierten Ultraschall empfängt und in ein elektrisches Signal umwandelt, das einer Auswerteeinheit zugeführt wird.

Aufgrund der unterschiedlichen Schallgeschwindigkeit in Fettgewebe und Muskelgewebe tritt an der Grenzschicht zwischen dem oberflächennahen Weichgewebe und dem Muskelgewebe eine Teilreflexion des Ultraschalles auf. Durch das an dem Applikator vorgesehene Ultraschall-Empfängerelement kann dieser reflektierte Ultraschall empfangen werden. Aus dem empfangenen reflektierten Ultraschall kann dann der Abstand der Fett-/Muskelgewebe-Grenzschicht und der Körperoberfläche und damit die Dicke der angesaugten oberflächennahen Weichgewebeschicht bestimmt werden. Die Bestimmung der Lage dieser Grenzschicht in bezug auf die Körperoberfläche hat den Vorteil, daß die Tiefenkontrolle der mit der erfindungsgemäßen Vorrichtung durchgeführten Ultraschalltherapie weiter verbessert werden kann. Über die Tiefenbestimmung des Überganges zwischen Fettgewebe und Muskelgewebe kann die Leistung bzw. die Leistungsdichte und auch die Tiefe des Fokus des Ultraschalls im Körpergewebe nämlich so eingestellt werden, daß es zu keiner zellzerstörenden Wirkung im Muskelgewebe kommt.

Weiterhin ist es bevorzugt, wenn die Ultraschall-Erzeugungsvorrichtung zumindest ein Schwingerelement zur Erzeugung von Ultraschall im diagnostischen Leistungs- und Frequenzbereich im Puls-Echobetrieb aufweist.

Dies kann auch das genannte Ultraschall-Empfängerelement sein. Während, wie zuvor beschrieben, auch der reflektierte Ultraschall im therapeutischen Leistungs- und Frequenzbereich dazu verwendet werden kann, die Tiefe der zu behandelnden Weichgewebeschicht zu ermitteln, wird Ultraschall im diagnostischen Leistungs- und Frequenzbereich, d.h. Ultraschall mit relativ hoher Frequenz (≥ 1 MHz) und geringerer Leistung, an einer Gewebe-Grenzfläche stärker reflektiert als Ultraschall im therapeutischen Frequenzbereich und ermöglicht eine höhere Auflösung. Somit ist vorteilhafterweise noch die Möglichkeit einer Kontrolle der Ultraschalltherapie unter Ultraschallbeobachtung gegeben, ohne daß ein zusätzliches Ultraschall-Bildgerät notwendig ist. Puls-Echo-Betrieb bedeutet, daß die Anregung des Schwingerelementes gepulst erfolgt, so daß das Schwingerelement Ultraschallpulse aussendet, wobei das Schwingerelement in den Sendepausen als Empfängerelement zum Empfangen reflektierter Ultraschallpulse arbeitet. Durch diese Betriebsart wird der Vorteil erzielt, daß bereits ein Schwingerelement zum Senden und Empfangen von Ultraschall ausreichend ist, um ein "eindimensionales" Ultraschallbild (A-Bild) zu erhalten.

In einer weiteren bevorzugten Ausgestaltung ist an der Applikatorfläche zumindest ein temperaturerfassendes Element angeordnet.

Mittels des temperaturerfassenden Elementes, bspw. eines thermoelektrischen Elements, kann die Temperatur an der Körperoberfläche erfaßt werden. Durch diese Maßnahme kann die Kontrolle der Ultraschallbehandlung weiter verbessert werden, indem bei einer festgestellten Temperaturerhöhung an der Körperoberfläche, d.h. der Haut, die Leistung des Ultraschalls entsprechend angepaßt werden kann. Außerdem kann im Fall, daß eine Temperaturerhöhung festgestellt wird, wie vorhergehend beschrieben wurde, ein Fluid in den Koppelbereich zwischen der Körperoberfläche und der Applikatorfläche eingeleitet werden, um die Körperoberfläche zu kühlen.

In einer weiteren bevorzugten Ausgestaltung weist der Applikator zumindest einen Punktionskanal auf.

Durch diese Maßnahme wird der Vorteil erzielt, daß über eine in den Punktionskanal eingebrachte Punktionsnadel durch den Ultraschall desintegriertes Weichgewebe, ggf. unter Ultraschallbildkontrolle, entfernt bzw. abgesaugt werden kann.

Weiterhin ist es bevorzugt, wenn in dem Applikator applikatorflächenseitig mindestens eine Elektrode zum Messen des Übergangswiderstandes zwischen dem Applikator und der Hautoberfläche angeordnet ist.

Mittels der mindestens einen Elektrode kann in Verbindung mit einer geeigneten elektrischen Quelle lokal der Übergangswiderstand zwischen dem Applikator und der Hautoberfläche gemessen werden. Sind mehrere Elektroden vorgesehen, können auch lokale Übergangswiderstände zwischen einzelnen Bereichen des Applikators und den entsprechenden Bereichen der Hautoberfläche gemessen werden, wodurch Bereiche der Hautoberfläche registriert werden können, die keinen optimalen Kontakt mit dem Applikator haben. In einem solchen Fall kann dann die Saugwirkung erhöht werden, um eine gleichmäßige Ansaugung der Hautoberfläche und des darunterliegenden oberflächennahen Weichgewebes zu erzielen. Es kann aber auch die über diesen Bereich der Hautoberfläche eingekoppelte Ultraschall-Leistung reduziert werden oder bspw. auch ein Warnsignal abgegeben werden, um ein Neuansetzen des Applikators zu empfehlen oder zu erzwingen. Somit können lokale Überwärmungen der Haut vermieden werden. Die Tiefenkontrolle der erfindungsgemäßen Vorrichtung wird durch diese Weiterbildung auf vorteilhafte Weise weiter verbessert. Vorteilhaft ist es dabei, wenn eine Vielzahl von Elektroden zwischen den Schwingerelementen angeordnet sind, so daß lokale Messungen des Übergangswiderstandes über den gesamten therapeutisch aktiven Bereich des Applikators möglich sind. Die für die Messung erforderliche Referenzelektrode ist dabei bevorzugt randseitig des Applikators, bspw. umfänglich an dem Rand des Applikators, angeordnet, kann aber auch direkt am Patienten angebracht sein.

Natürlich kann die erfindungsgemäße Vorrichtung auch ggf. in miniaturisierter Form, insbesondere endoskopisch, zur Therapie eingesetzt werden.

Weitere Vorteile ergeben sich aus der nachfolgenden Beschreibung der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen bzw. in Alleinstellung verwendbar sind, ohne den Rahmen der vorliegenden Erfindung zu verlassen.

Ausführungsbeispiele der erfindung sind in der Zeichnung dargestellt und werden hiernach mit Bezug auf die Zeichnung näher erläutert. Es zeigen:
- Fig. 1: schematisch eine Vorrichtung zur Behandlung von Körpergewebe teilweise im Längsschnitt;
- Fig. 2: eine Vorderansicht eines Applikators der Vorrichtung in Fig. 1;
- Fig. 3: die Vorrichtung in Fig. 1 im Ausschnitt während der Behandlung des Körpergewebes; und
- Fig. 4: ein zweites Ausführungsbeispiel eines Applikators der Vorrichtung in Fig. 1 in perspektivischer Darstellung.

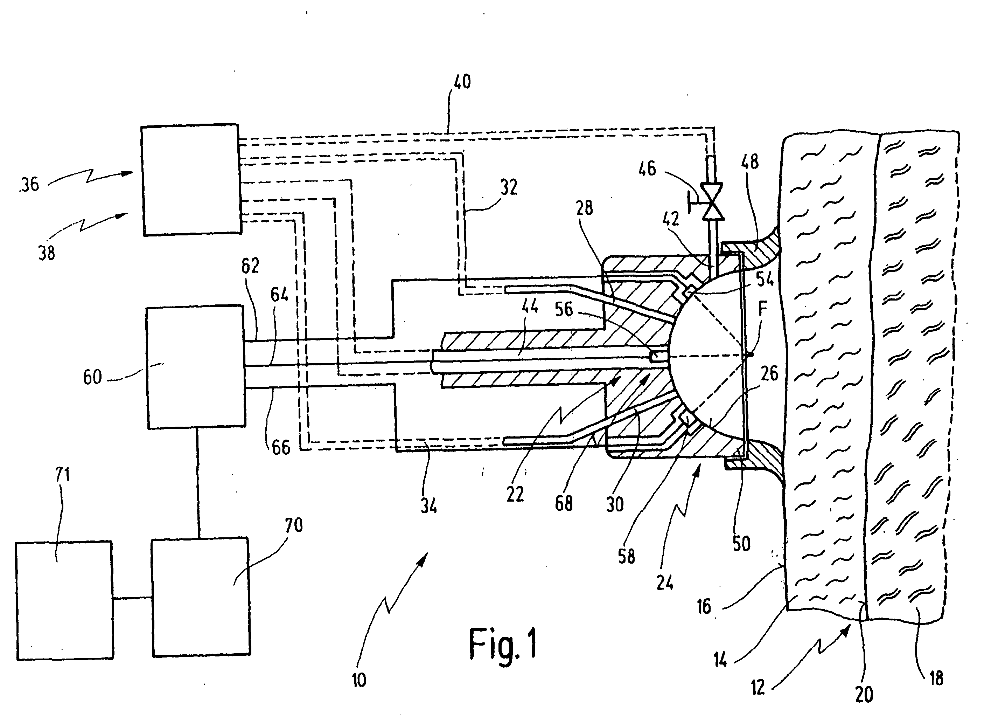

In Fig. 1 ist eine mit dem allgemeinen Bezugszeichen 10 versehene Vorrichtung zur Behandlung von Körpergewebe 12 mittels Ultraschall dargestellt. Das zu behandelnde Körpergewebe 12 ist ein oberflächennahes Weichgewebe 14, bestehend aus den Schichten Epidermis, Dermis und Fettgewebe, das sich zwischen der Körperoberfläche 16, d.h. der Hautoberfläche, und einem sich an das oberflächennahe Weichgewebe 14 anschließenden Muskelgewebe 18 befindet. Zwischen dem oberflächennahen Weichgewebe 14 und dem Muskelgewebe 18 ist in Fig. 1 eine Gewebsgrenzfläche 20 zur Veranschaulichung dargestellt.

Die Vorrichtung 10 weist eine Ultraschall-Erzeugungseinheit 22 und einen Applikator 24 auf. Mittels des Applikators 24 wird der Ultraschall von außen durch die Körperoberfläche 16 in das darunterliegende Körpergewebe 12 eingekoppelt.

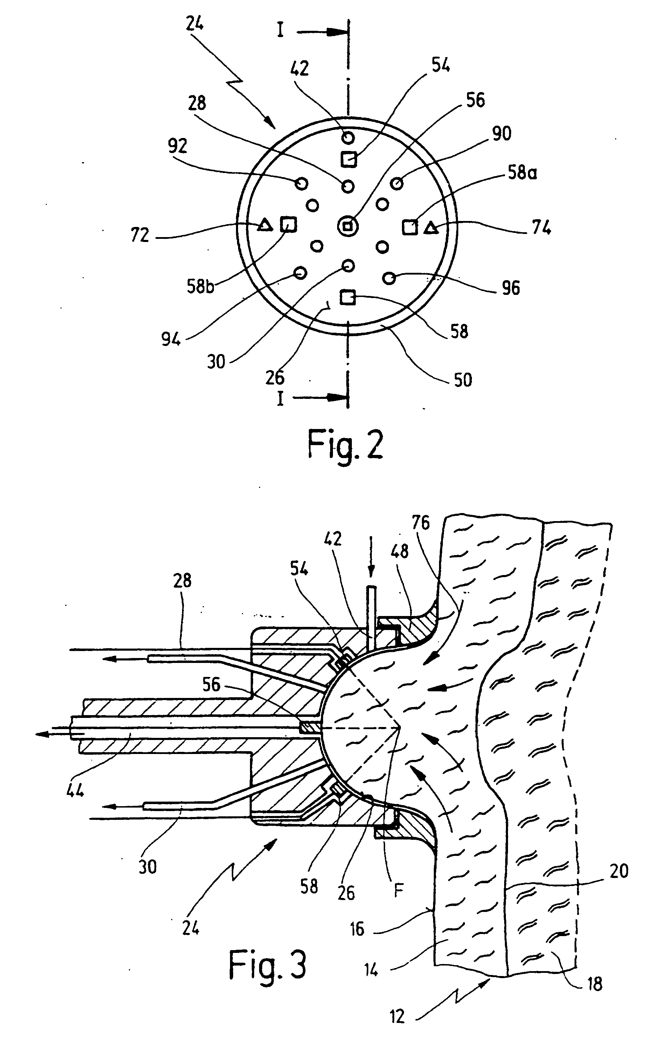

Eine der Körperoberfläche 16 zugewandte Applikatorfläche 26 ist nach innen gewölbt ausgebildet. Die Applikatorfläche 26 weist in dem in Fig. 1 und 2 dargestellten Ausführungsbeispiel eine etwa radialsymmetrisch kalottenförmige Auswölbung auf.

Erfindungsgemäß ist für die Vorrichtung 10 eine Ansaugvorrichtung zum Ansaugen der Körperoberfläche 16 an den Applikator 24 vorgesehen, wie hiernach beschrieben wird.

In dem Applikator 24 sind dazu Saugkanäle 28 und 30 ausgebildet, die in die Applikatorfläche 26 münden. In dem in Fig. 1 und 2 dargestellten Ausführungsbeispiel münden insgesamt sechs Saugkanäle 28, 30 radialsymmetrisch verteilt in der Applikatoroberfläche 26. Die Saugkanäle 28, 30 stehen über Verbindungsleitungen 32 und 34 mit einer Saugvorrichtung 36 in Verbindung. Mittels der Saugvorrichtung 36 können die Saugkanäle 28, 30 mit Unterdruck beaufschlagt werden.

Die Saugvorrichtung 36 enthält integriert ferner eine Saug- und Spülvorrichtung 38 für ein Fluid. Das Fluid kann über eine Verbindungsleitung 40 und einen randseitig der Applikatorfläche 26 mündenden Einlaßkanal 42 in den Bereich zwischen der Körperoberfläche 16 und der Applikatorfläche 26 eingelassen werden und über einen in der Applikatorfläche 26 mittig mündenden Absaugkanal 44 wieder abgesaugt werden.

In der Verbindungsleitung 40 zwischen der Saug- und Spülvorrichtung 38 ist ein Mittel 46 zum Absperren oder Drosseln der Fluidzufuhr in den Einlaßkanal 42 angeordnet, bspw. ein Absperrventil oder ein Regelventil zum Einstellen eines Unterdrucks in dem Raum zwischen der Applikatorfläche 26 und der Körperoberfläche 16.

An einem Rand 50 des Applikators 24 ist ein Abstandshalter 48 als Distanzstück zwischen der Körperoberfläche 16 und dem Applikator 24 befestigbar. Der Abstandshalter 48 ist bspw. eine Manschette aus einem elastischen Material. Der Abstandshalter 48 dient gleichzeitig als Abdichtung des Applikators 24 gegenüber der Körperoberfläche 16. Ohne den Abstandshalter 48 kann eine Abdichtung des Applikators 24 gegen die Körperoberfläche 16 durch eine elastische Ausgestaltung des Randes 50 des Applikators 24 oder durch Aufbringen eines Dichtungsmaterials auf die Körperoberfläche 16 im Bereich des Randes 50 erreicht werden. Es können für den Applikator 24 mehrere Abstandshalter 48 für unterschiedliche Abstandseinstellungen bereitstehen.

Die Ultraschall-Erzeugungseinheit 22 weist mehrere, vorzugsweise symmetrisch angeordnete, Schwingerelemente 54, 56, 58, 58a und 58b auf. In dem in Fig. 1 und Fig. 2 dargestellten Ausführungsbeispiel sind insgesamt fünf Schwingerelemente 54, 56, 58, 58a, 58b vorgesehen. Die Schwingerelemente 54, 56, 58, 58a, 58b sind elektrisch anregbare Ultraschall-Wandler, bspw. in Form von piezo-keramischen Elementen. Zur Anregung der Schwingerelemente 54, 56, 58, 58a, 58b ist eine Steuereinheit 60 vorgesehen, mit der die Schwingerelemente 54, 56, 58, 58a, 58b über elektrische Verbindungsleitungen 62, 64 und 66 verbunden sind.

Die Schwingerelemente 54, 56, 5-8, 58a, 58b sind in der Applikatorfläche 26 angeordnet. Aufgrund der etwa radialsymmetrisch kalottenförmigen Ausbildung der Applikatorfläche 26 wird der von den Schwingerelementen 54, 56, 58, 58a, 58b emittierte Ultraschall auf einen eng begrenzten Fokus-Bereich F fokussiert. Es können auch mehr als fünf Schwingerelemente 54, 56, 58, 58a, 58b vorgesehen sein, die in der Applikatorfläche 26 verteilt angeordnet sind bzw. die Applikatorfläche 26 weitgehend bedekken können, deren ausgesendeter Ultraschall im Fokus-Bereich F konzentriert ist. Der Fokus-Bereich F weist einen Abstand vom Scheitelpunkt der Applikatorfläche 26 in einem Bereich von 5 bis 40 mm auf.

Die Schwingerelemente 54, 56, 58, 58a, 58b werden von der Steuereinheit 60 gepulst oder kontinuierlich angeregt, je nachdem, ob das Körpergewebe 12 mit einem gepulsten oder kontinuierlichen Ultraschallwellenfeld behandelt werden soll.

Es ist auch möglich, die Schwingerelemente 54, 56, 58, 58a, 58b mit unterschiedlichen Phasen bzw. unterschiedlichen Frequenzen anzuregen. Das Schwingerelement 54 kann dazu bspw. mit der doppelten Frequenz wie das Schwingerelement 58 angeregt werden. Auch ist es möglich, den Fokus-Bereich F durch eine entsprechende Ansteuerung der Schwingerelemente 54, 56, 58, 58a, 58b über die Steuereinheit 60 in seiner Lage und in seiner Ausdehnung zu verändern.

Ferner ist in der Applikatorfläche 26 ein Ultraschall-Empfängerelement 68 angeordnet, das reflektierten Ultraschall in elektrische Signale umwandeln kann, die einer Auswerteeinheit 70 zugeführt werden, in der die empfangenen Signale ausgewertet werden. Bei dem reflektierten Ultraschall handelt es sich um am Übergang von der Körperoberfläche 16, d.h. der Hautoberfläche, zu dem oberflächennahen Weichgewebe 14 und um an der Gewebsgrenzfläche 20 zwischen dem oberflächennahen Weichgewebe 14 und dem Muskelgewebe 18 reflektierten Ultraschall. Aus den empfangenen reflektierten Ultraschallsignalen kann der Abstand der Gewebsgrenzfläche 20 von der Körperoberfläche 16 bestimmt werden. Anhand der ausgewerteten Ergebnisse kann dann die Lage des Fokus-Bereichs F wie gewünscht eingestellt werden.

Anstatt ein Schall-Empfängerelement 68 in der Applikatorfläche 26 vorzusehen, ist es auch möglich, das mittig in der Applikatorfläche 26 angeordnete Schwingerelement 56 zur Bestimmung der Lage der Grenzgewebsschicht 20 vorzusehen. Dazu wird das Schwingerelement 56 gepulst angeregte um Ultraschall im diagnostischen Leistungsbereich zu emittieren, wobei das Schwingerelement 56 dann in den Sendepausen reflektierten Ultraschall empfängt und den Ultraschall in ein elektrisches Signal umwandelt, das der Auswerteeinheit 70 über die Steuereinheit 60 zugeführt wird. Der Auswerteeinheit 70 ist dazu noch eine Ultraschall-Bildgebungseinheit 71 zugeordnet.

Ferner sind in dem Applikator 24 temperaturerfassende Elemente 72 und 74 angeordnet. Die temperaturerfassenden Elemente 72 und 74, bspw. thermoelektrische. Elemente, sind im Randbereich der Applikatorfläche 26 angeordnet. Mit den temperaturerfassenden Elementen 72 und 74 kann die Temperatur der Körperoberfläche 16 während der Behandlung des Weichgewebes 14 mit Ultraschall überwacht werden. Hierzu können die temperaturerfassenden Elemente 72 und 74 über nicht dargestellte Verbindungsleitungen mit der Steuereinheit 60 oder der Auswerteeinheit 70 verbunden sein.

In Fig. 3 ist der Applikator 24 während der Behandlung des Körpergewebes 12 dargestellt. Indem die Saugleitungen 28, 30 über die Saugvorrichtung 36 mit Unterdruck beaufschlagt werden, wird die Körperoberfläche 16 und damit das Weichgewebe 14 in die Auswölbung des Applikators 24 gesaugt. Aufgrund der Nachgiebigkeit des oberflächennahen Weichgewebes 14 wird dieses stärker angesaugt als das härtere Muskelgewebe 18. Mit Pfeilen 76 ist veranschaulicht, daß auch außerhalb des Umfanges des Applikators 24 vorhandenes oberflächennahes Weichgewebe 14 verstärkt in die Auswölbung gesaugt wird. Durch Schwingungsanregung der Schwingerelemente 54, 56, 58, 58a, 58b wird nun Ultraschall im therapeutischen Leistungs- und Frequenzbereich im Fokus-Bereich F fokussiert in das Weichgewebe 14 eingekoppelt.

Vor dem Ansaugen des Weichgewebes 14 kann der Koppelbereich zwischen der Körperoberfläche 16 und der Applikatorfläche 26 mit dem Fluid gefüllt werden, indem das Fluid von der Spül- und Saugvorrichtung 38 über den Einlaßkanal 42 eingeleitet wird. Das Fluid kann dann über den Absaugkanal 44 bei geschlossenem Mittel 46 zum Absperren der Verbindungsleitung 40 abgesaugt werden, wodurch gleichzeitig die Körperoberfläche 16 in die Auswölbung des Applikators 24 gesaugt wird. Eine Fluidschicht verbleibt dann zwischen der Körperoberfläche 16 und der Applikatorfläche 26, wodurch die Körperoberfläche 16, d.h. die Haut, während der Ultraschallbehandlung gekühlt wird. Wenn das Mittel 46 die Verbindungsleitung 40 nicht vollständig absperrt, ist auch weiterhin ein begrenztes Fließen des Fluids möglich, so daß eine besonders effektive Kühlung stattfindet. Während der Behandlung des Weichgewebes 14 kann der Fokus-Bereich F nach einer wie zuvor beschriebenen Bestimmung der Lage der Gewebsgrenzfläche 20 entsprechend verstellt und angepaßt werden. Die Tiefe des Fokus in dem Weichgewebe 15 wird auf etwa die Hälfte der Dicke des oberflächennahen Weichgewebes 14 bzw. der Fettschicht eingestellt. Während der Ultraschallbehandlung wird die Temperatur der Körperoberfläche 16 mittels der temperaturerfassenden Elemente 72 und 74 erfaßt. Aus der erfaßten Temperatur kann eine weitere Anpassung der Leistung des eingekoppelten Ultraschalls erfolgen. Die Therapie kann unter In-Line-Ultraschallbildkontrolle mittels der Ultraschall-Bildgebungseinheit 71 durchgeführt werden.

Die Effekte, die durch therapeutischen, d.h. Leistungsultraschall am Weichgewebe 14 bewirkt werden, sind zum einen Erwärmung und zum anderen durch Kavitation ausgelöste mechanische Desintegration. Durch die beschriebene Einstellbarkeit von Ultraschall-Leistung und -Frequenz und Ausdehnung des Fokus-Bereichs F kann der jeweils gewünschte Effekt bewirkt werden. Bei einer Tumorbehandlung wird die Lokalerwärmung des Körpergewebes 12 durch den fokussierten Ultraschall ausgenutzt. Bei der Desintegration des Weichgewebes 14 spielt der durch den Ultraschall bewirkte Effekt der Kavitation eine größere Rolle. Durch die Expansion und das Platzen von Kavitationsblasen werden Zellverbände gelöst. Die Schwellwerte zur Bildung von Kavitation liegen bei einer Frequenz der Schwingerelemente von 750 kHz bis 3,5 MHz. Ebenso begünstigt ein Pulsbetrieb der Schwingerelemente 54, 56, 58, 58a, 58b die Kavitationsbildung stärker als ein kontinuierlicher Betrieb der Schwingerelemente 54, 56, 58, 58a, 58b. Außerdem führt der Pulsbetrieb zu einer geringeren Gewebeerwärmung im Fokus-Bereich F bzw. auf der Körperoberfläche 16. Die Schwellwerte zur Bildung von Kavitation im Körpergewebe 12 können signifikant erniedrigt werden, wenn Mikroblasen im Körpergewebe 12 vorhanden sind bzw. in das zu behandelnde Körpergewebe 12 eingebracht werden. Im Falle des oberflächennahen Weichgewebe 14, wie Epidermis, Dermis, Fettgewebe, ist es möglich, Mikroblasen nebenwirkungsfrei einzubringen. Als Kavitationsbeschleuniger eignen sich in erster Linie Ultraschallkontrastmittel mit Mikroblasen, welche bisher zur verbesserten Ultraschall-Darstellung von Gefäßen eingesetzt werden.

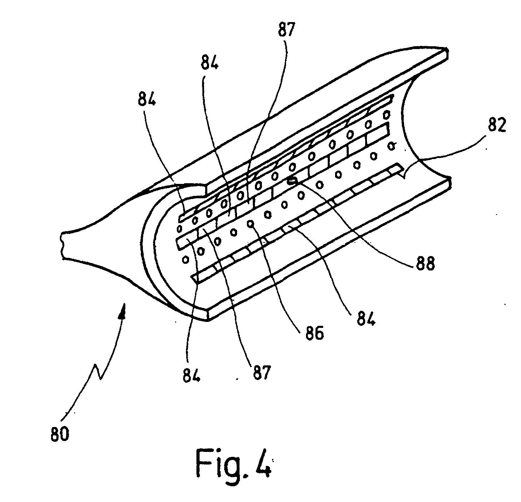

In Fig. 4 ist als weiteres Ausführungsbeispiel ein Applikator 80 dargestellt, der in der Vorrichtung 10 in Fig. 1 eingesetzt werden kann. Der Applikator 80 weist eine nach innen gewölbte Applikatorfläche 82 auf. Die Seitenteile sind der besseren Übersicht halber nicht dargestellt. Die Applikatorfläche 82 ist zylindersymmetrisch mit etwa U-förmigem Querschnitt ausgebildet. In der Applikatorfläche 82 ist eine Mehrzahl von Schwingerelementen 84 in Form von Linienarrays angeordnet. Mit einer derartigen Ausgestaltung des Applikators 80 wird der von den Schwingerelementen 84 abgestrahlte Ultraschall nicht auf einen punktförmigen, sondern einen linienförmigen Fokus-Bereich konzentriert. Die Arrays der Schwingerelemente 84 werden bevorzugt so angesteuert, daß der Fokus über die gesamte Länge der Applikatorfläche 82 einen konstanten Abstand von dem Scheitelpunkt der Applikatorfläche aufweist.

Weiterhin münden in die Applikatorfläche 82 ein oder mehrere Saugkanäle 86 zum Ansaugen des Körpergewebes 12, die auch in Form zur Applikatorfläche 82 offener Rillen miteinander verbunden sein können. Es versteht sich, daß die im Zusammenhang mit dem Applikator 24 erläuterten Merkmale, wie bspw. die Möglichkeit einer Fluidzufuhr und -absaugung auch bei dem Applikator 80 vorgesehen sind, ohne daß es einer näheren Erläuterung bedarf.

Weiterhin kann ein zweites Array von Schwingerelementen 87 für Puls-Echo-Betrieb im diagnostischen Bereich in Längsrichtung des Applikators 80 vorgesehen sein, mit dem ein Ultraschall-B-Bild zur Kontrolle der Therapie erfaßt werden kann. Die Schwingerelemente 84 und 87 können bspw. auf einer Linie abwechselnd in der Applikatorfläche 82 angeordnet sein.

Weiterhin sind bei dem in Fig. 1 dargestellten Applikator 24 und bei dem in Fig. 4 dargestellten Applikator 80 abdichtbare Punktionskanäle bspw. mittig in der Applikatorfläche vorgesehen, wie bei dem Applikator 80 mit dem Bezugszeichen 88 dargestellt ist, um desintegriertes Weichgewebe 14 unter Ultraschallbildkontrolle über eine eingebrachte Punktionsnadel zu entfernen.

In Fig 2 sind in dem Applikator 24 in der Applikatorfläche 26 Elektroden 90, 92, 94, 96 dargestellt, mit denen lokal ein Übergangswiderstand zwischen der Applikatorfläche 26 und der Hautoberfläche 16 gemessen werden kann, um die Saugwirkung zu kontrollieren und in dem Fall, daß an einer Stelle der Hautoberfläche ein unzureichender Kontakt mit der Applikatorfläche festgestellt wird, die Saugwirkung über die Saugleistung der Saugvorrichtung 36 entsprechend einzustellen. Die Elektroden 90, 92, 94, 96 sind zwischen den Schwingerelementen 54, 56, 58, 58a, 58b verteilt angeordnet und können auch in größerer Anzahl als vier vorgesehen sein, um eine flächendeckende Messung zu ermöglichen.

Die Elektroden 90, 92, 94, 96 sind mit einer hier nicht dargestellten geeigneten elektrischen Quelle verbunden.

Eine hier nicht dargestellte Referenzelektrode ist an dem Applikator 24 randseitig, bspw. umfänglich an dem Rand 50 des Applikators 24 angeordnet oder direkt an dem Patienten angebracht.

## Patentansprüche

1. Vorrichtung zur Behandlung von Körpergewebe (12), insbesondere von oberflächennahem Weichgewebe (14), mittels Ultraschall, mit einer Ultraschall-Erzeugungseinheit (22) und mit einem Applikator (24; 80), mit dem der Ultraschall über eine der Körperoberfläche (16) zugewandte Applikatorfläche (26; 82) von außen durch die Körperoberfläche (16) in das Körpergewebe (12) einkoppelbar ist, und mit einer Ansaugvorrichtung zum Ansaugen der Körperoberfläche (16) an den Applikator (24; 80), wobei die Ansaugvorrichtung eine Saugvorrichtung (36) aufweist, mit der der Applikator (24; 80) über zumindest einen Saugkanal (28, 30; 86) in Verbindung steht, und wobei die Applikatorfläche (26; 82) nach innen gewölbt ausgebildet ist, und wobei die Auswölbung der Applikatorfläche (26; 82) nach innen zum Ansaugen der Körperoberfläche (16) bis an die Applikatorfläche (26; 82) dient, **dadurch gekennzeichnet, daß** der zumindest eine Saugkanal (28, 30; 86) in der Applikatorfläche (26; 82) mündet.

2. Vorrichtung nach Anspruch 1, **dadurch gekennzeichnet, daß** der Ultraschall mittels des Applikators (24; 80) fokussiert in das Körpergewebe (12) einkoppelbar ist.

3. Vorrichtung nach Anspruch 1 oder 2, **dadurch gekennzeichnet, daß** die Applikatorfläche (26) etwa radialsymmetrisch kalottenförmig ausgebildet ist.

4. Vorrichtung nach Anspruch 1 oder 2, **dadurch gekennzeichnet, daß** die Applikatorfläche (82) etwa zylindersymmetrisch mit etwa U-förmigem Querschnitt ausgebildet ist.

5. Vorrichtung nach einem der Ansprüche 1 bis 4, **dadurch gekennzeichnet, daß** die Ultraschall-Erzeugungseinheit (22) ein elektrisch anregbares Schwingerelement (56; 84) oder mehrere elektrisch anregbare Schwingerelemente (54, 56, 58; 84) aufweist, das in der Applikatorfläche (26; 82) angeordnet ist bzw. die in der Applikatorfläche (26; 82) angeordnet sind.

6. Vorrichtung nach Anspruch 5, **dadurch gekennzeichnet, daß** die Schwingerelemente (54, 56, 58; 84) mit unterschiedlichen Frequenzen und/oder mit unterschiedlichen Phasen anregbar sind.

7. Vorrichtung nach einem der Ansprüche 1 bis 6, **dadurch gekennzeichnet, daß** der Applikator (24; 80) mit einer Saug- und Spülvorrichtung (38) für ein Fluid in Verbindung steht, das zwischen die Applikatorfläche (26; 82) und die Körperoberfläche (16) gebracht und wieder abgesaugt werden kann.

8. Vorrichtung nach Anspruch 7, **dadurch gekennzeichnet, daß** ein Absaugkanal (44) für das Fluid mittig in der Applikatorfläche (26; 82) mündet.

9. Vorrichtung nach Anspruch 7 oder 8, **dadurch gekennzeichnet, daß** mindestens ein Einlaßkanal (42) randseitig des Applikators (24; 80) in den Bereich zwischen die Applikatorfläche (26; 82) und die Körperoberfläche (16) mündet.

10. Vorrichtung nach einem der Ansprüche 1 bis 9, **dadurch gekennzeichnet, daß** der Applikator (24; 80) randseitig eine Abdichtung gegenüber der Körperoberfläche (16) aufweist.

11. Vorrichtung nach einem der Ansprüche 1 bis 10, **dadurch gekennzeichnet, daß** die Tiefe des Fokus des Ultraschalls im Körpergewebe (12) einstellbar ist.

12. Vorrichtung nach einem der Ansprüche 5 bis 11, **dadurch gekennzeichnet, daß** der Abstand zwischen dem bzw. den Schwingerelementen (54, 56, 58; 84) und der Körperoberfläche (16) verstellbar ist.

13. Vorrichtung nach einem der Ansprüche 1 bis 12, **dadurch gekennzeichnet, daß** an dem Applikator (24; 80) ein Abstandshalter (48) befestigbar ist.

14. Vorrichtung nach einem der Ansprüche 1 bis 13, **dadurch gekennzeichnet, daß** der Abstand des Fokus von der Applikatorfläche (26; 82) zwischen 5 und 40 mm beträgt.

15. Vorrichtung nach einem der Ansprüche 1 bis 14, **dadurch gekennzeichnet, daß** der Applikator (24; 80) zumindest ein Ultraschall-Empfängerelement (68) aufweist, das reflektierten Ultraschall empfängt und in ein elektrisches Signal umwandelt, das einer Auswerteeinheit (70) zugeführt wird.

16. Vorrichtung nach einem der Ansprüche 1 bis 15, **dadurch gekennzeichnet, daß** die Ultraschall-Erzeugungsvorrichtung (22) zumindest ein Schwingerelement zur Erzeugung von Ultraschall im diagnostischen Leistungs- und Frequenzbereich im Puls-Echo-Betrieb aufweist.

17. Vorrichtung nach einem der Ansprüche 1 bis 16, **dadurch gekennzeichnet, daß** an der Applikatorfläche (26; 82) zumindest ein temperaturerfassendes Element (72, 74) angeordnet ist.

18. Vorrichtung nach einem der Ansprüche 1 bis 15, **dadurch gekennzeichnet, daß** der Applikator (24; 80) zumindest einen Punktionskanal aufweist.

19. Vorrichtung nach einem der Ansprüche 1 bis 18, **dadurch gekennzeichnet, daß** in dem Applikator (24; 80) applikatorflächenseitig mindestens eine Elektrode (90, 92, 94, 96) zum Messen des Übergangswiderstandes zwischen dem Applikator (24; 80) und der Hautoberfläche (16) angeordnet ist.

20. Vorrichtung nach einem der Ansprüche 1 bis 19, **dadurch gekennzeichnet, daß** sie für den endoskopischen Einsatz ausgebildet ist.

## Claims

1. An apparatus for treating body tissue (12), in particular superficial soft tissue (14), with ultrasound, comprising an ultrasonic generation unit (22) and an applicator (24; 80), by means of which the ultrasound can be irradiated from an applicator face (26; 82) facing the body surface (16) from outside through the body surface (16) into the body tissue (12), further comprising a suction apparatus for taking in the body surface (16) against the applicator (24; 80), wherein the suction apparatus comprises an aspiration apparatus (36) with which the applicator (24; 80) is connected via at least one suction channel (28, 30; 86), and wherein the applicator face (26; 82) is curved inwardly, and wherein the inwardly directed curvature serves for taking in the bodysurface (16) up to the applicator face (26; 82), **characterized in that** the at least one suction channel (28, 30; 86) opens into the applicator face (26; 82).

2. The apparatus of claim 1, **characterized in that** the ultrasound can be irradiated by means of the applicator (24; 80) into the body tissue (12) in a focused manner.

3. The apparatus of claim 1 or 2, **characterized in that** the applicator face (26) has an approximately radial symmetric spherical cap shape.

4. The apparatus of claim 1 or 2, **characterized in that** the applicator face (82) has a cylindric symmetric shape with an approximately U-shaped cross-section.

5. The apparatus of anyone of claims 1 through 4, **characterized in that** the ultrasonic generation unit (22) comprises an electrically excitable transducer element (56; 84) disposed in the applicator face (26; 82), or a plurality of electrically excitable transducer elements (54, 56, 58; 84) disposed in the applicator face (26; 82).

6. The apparatus of claim 5, **characterized in that** the transducer elements (54, 56, 58; 84) are excitable with different frequencies and/or with different phases.

7. The apparatus of anyone of claims 1 through 6, **characterized in that** the applicator (24; 80) is combined with a suction and irrigation apparatus (38) for a fluid which fluid can be brought between the applicator face (26; 82) and the body surface (16) and be sucked off again.

8. The apparatus of claim 7, **characterized in that** a suction channel (44) for the fluid opens into the applicator face (26; 82) in the center thereof.

9. The apparatus of claim 7 or 8, **characterized in that** at least one inlet channel (42) opens at the periphery of the applicator (24; 80) into the region between the applicator face (26; 82) and the body surface.

10. The apparatus of anyone of claims 1 through 9, **characterized in that** the applicator (24; 80) comprises at the periphery thereof a sealing towards the body surface (16).

11. The apparatus of anyone of claims 1 through 10, **characterized in that** the depth of focus of the ultrasound in the body tissue (12) is adjustable.

12. The apparatus of anyone of claims 5 through 11, **characterized in that** the distance between the transducer element or elements (54, 56, 58; 84) and the body surface (16) is adjustable.

13. The apparatus of anyone of claims 1 through 12, **characterized in that** a spacer is attachable to the applicator (24; 80).

14. The apparatus of anyone of claims 1 through 13, **characterized in that** the distance between the focus and the applicator face (26; 82) is within the range of 5 and 40 mm.

15. The apparatus of anyone of claims 1 through 14, **characterized in that** the applicator (24; 80) comprises at least one ultrasonic receiver element (68) receiving reflected ultrasound and converting same into an electric signal, which is fed to an evaluation unit (70).

16. The apparatus of anyone of claims 1 through 15, **characterized in that** the ultrasonic generation unit (22) comprises at least one transducer element for generating ultrasound in the diagnostic power and frequency range in pulse-echo mode operation.

17. The apparatus of anyone of claims 1 through 16, **characterized in that** at least one temperature registrating element (72, 74) is disposed at the applicator face (26; 82).

18. The apparatus of anyone of claims 1 through 15, **characterized in that** the applicator (24; 80) comprises at least one puncture channel.

19. The apparatus of anyone of claims 1 through 18, **characterized in that** at least one electrode (90, 92, 94, 96) for measuring the transition resistance between the applicator (24; 80) and the body surface (16) is disposed in the applicator (24; 80) at the applicator face thereof.

20. The apparatus of anyone of claims 1 through 19, **characterized in that** it is configured for endoscopic use.

## Revendications

1. Dispositif de traitement par ultrasons des tissus physiologiques (12), en particulier les tissus mous (14) à proximité de la surface, comportant une unité de génération d'ultrasons (22) et un applicateur (24 ; 80) par lequel les ultrasons peuvent être injectés de l'extérieur, par l'intermédiaire d'une face d'application (26 ; 82), orientée vers la surface du corps (16), à travers la surface du corps (16) dans le tissu physiologique (12), et comportant un dispositif de succion destiné à attirer la surface du corps (16) contre l'applicateur (24 ; 80), le dispositif de succion comportant un dispositif d'aspiration (38) avec lequel communique l'applicateur (24 ; 80) via au moins un canal d'aspiration (28, 30 ; 86), et la face d'application (26 ; 82) étant conformée de façon concave vers l'intérieur et la concavité de la face d'application (26 ; 82) vers l'intérieur étant destinée à attirer la surface du corps (16) jusque contre la face d'application (26 ; 82), **caractérisé en ce que** ledit au moins un canal d'aspiration (28, 30 ; 86) débouche dans la face d'application (26 ; 82).

2. Dispositif selon la revendication 1, **caractérisé en ce que** les ultrasons, au moyen de l'applicateur (24 ; 80), sont injectés sous forme focalisée dans le tissu physiologique (12).

3. Dispositif selon la revendication 1 ou 2, **caractérisé en ce que** la face d'application (26) est réalisée sous forme de calotte à peu près radialement symétrique.

4. Dispositif selon la revendication 1 ou 2, **caractérisé en ce que** la face d'application (82) est réalisée avec une section sensiblement en forme de U avec approximativement une symétrie cylindrique.

5. Dispositif selon l'une des revendications 1 à 4, **caractérisé en ce que** l'unité de génération des ultrasons (22) comporte un oscillateur (56 ; 84) pouvant être excité électriquement, ou plusieurs oscillateurs (54, 56, 58 ; 84) pouvant être excités électriquement, qui est disposé dans la face d'application (26 ; 82) ou qui sont disposés dans la face d'application (26 ; 82).

6. Dispositif selon la revendication 5, **caractérisé en ce que** les oscillateurs (54, 56, 58 ; 84) peuvent être excités avec différentes fréquences et/ou avec différentes phases.

7. Dispositif selon l'une des revendications 1 à 6, **caractérisé en ce que** l'applicateur (24 ; 80) communique avec un dispositif d'aspiration et de refoulement (38) pour un fluide, qui peut être amené entre la face d'application (26 ; 82) et la surface du corps (16) et être à nouveau évacué par aspiration.

8. Dispositif selon la revendication 7, **caractérisé en ce qu'**un canal d'évacuation (44) pour le fluide débouche au milieu de la face d'application (26 ; 82).

9. Dispositif selon la revendication 7 ou 8, **caractérisé en ce qu'**au moins un canal d'admission (42) débouche du côté du bord de l'applicateur (24 ; 80), dans la zone entre la face d'application (26 ; 82) et la surface du corps (16).

10. Dispositif selon l'une des revendications 1 à 9, **caractérisé en ce que** l'applicateur (24 ; 80) comporte sur son bord une garniture d'étanchéité par rapport à la surface du corps (16).

11. Dispositif selon l'une des revendications 1 à 10, **caractérisé en ce que** la profondeur du foyer du faisceau ultrasonore dans le tissu physiologique (12) est réglable.

12. Dispositif selon l'une des revendications 5 à 11, **caractérisé en ce que** la distance entre l'oscillateur ou les oscillateurs (54, 56, 58 ; 84) et la surface du corps (16) est réglable.

13. Dispositif selon l'une des revendications 1 à 12, **caractérisé en ce qu'**un écarteur (48) peut être fixé sur l'applicateur (24 ; 80).

14. Dispositif selon l'une des revendications 1 à 13, **caractérisé en ce que** la distance entre le foyer et la face d'application (26 ; 82) se situe entre 5 et 40 mm.

15. Dispositif selon l'une des revendications 1 à 14, **caractérisé en ce que** l'applicateur (24 ; 80) comporte au moins un élément récepteur d'ultrasons (68), qui reçoit le faisceau ultrasonore réfléchi et le transforme en signal électrique, lequel est acheminé vers une unité d'analyse (70).

16. Dispositif selon l'une des revendications 1 à 15, **caractérisé en ce que** le dispositif de génération d'ultrasons (22) comporte au moins un oscillateur destiné à générer des ultrasons dans la zone de puissance et de fréquence de diagnostic en mode de fonctionnement à impulsions réfléchies.

17. Dispositif selon l'une des revendications 1 à 16, **caractérisé en ce qu'**au moins un élément (72, 74) enregistrant la température est disposé sur la face d'application (26 ; 82).

18. Dispositif selon l'une des revendications 1 à 15, **caractérisé en ce que** l'applicateur (24 ; 80) comporte au moins un canal de ponction.

19. Dispositif selon l'une des revendications 1 à 18, **caractérisé en ce qu'**au moins une électrode (90, 92, 94, 96), destinée à mesurer la résistance de contact entre l'applicateur (24 ; 80) et la surface de la peau (16), est disposée dans l'applicateur (24 ; 80) du côté de la face d'application.

20. Dispositif selon l'une des revendications 1 à 19, **caractérisé en ce qu'**il est conçu pour un usage endoscopique.
